# EUROPEAN PATENT SPECIFICATION

(11) **EP 3 340 089 B1**
(45) Date of publication and mention of the grant of the patent: **16.06.2021**
(21) Application number: 16205197.3
(22) Date of filing: 20.12.2016
(51) Int. Cl.: G16H 10/20, G16H 50/30

(54) **SYSTEMS, METHODS AND APPARATUSES FOR ESTIMATING THE STRESS VALENCE OF AN INDIVIDUAL FROM SENSOR DATA**
SYSTEME, VERFAHREN UND VORRICHTUNGEN ZUR SCHÄTZUNG DER BELASTUNGSVALENZ EINES INDIVIDUUMS AUS SENSORDATEN
SYSTÈMES, PROCÉDÉS ET APPAREILS PERMETTANT D'ESTIMER LA VALENCE DE TENSION D'UN INDIVIDU À PARTIR DE DONNÉES DE CAPTEUR

(43) Date of publication of application: 27.06.2018
(73) Proprietor: Nokia Technologies Oy, 02610 Espoo (FI)
(72) Inventor: BAILEY, Marc James Ashton, Cambridge, CB4 3DD (GB); RAHMAN, MD Mahbubur, Sunnyvale, CA 94086 (US)
(74) Representative: Whiting, Gary

(56) References cited:
- HILLOL SARKER ET AL: "Finding Significant Stress Episodes in a Discontinuous Time Series of Rapidly Varying Mobile Sensor Data", HUMAN FACTORS IN COMPUTING SYSTEMS, ACM, 2 PENN PLAZA, SUITE 701 NEW YORK NY 10121-0701 USA, 7 May 2016 (2016-05-07), pages 4489-4501, XP058257306, DOI: 10.1145/2858036.2858218 ISBN: 978-1-4503-3362-7
- HILLOL SARKER ET AL: "Assessing the availability of users to engage in just-in-time intervention in the natural environment", PERVASIVE AND UBIQUITOUS COMPUTING, ACM, 2 PENN PLAZA, SUITE 701 NEW YORK NY 10121-0701 USA, 13 September 2014 (2014-09-13), pages 909-920, XP058055186, DOI: 10.1145/2632048.2636082 ISBN: 978-1-4503-2968-2
- RUI WANG ET AL: "CrossCheck", PERVASIVE AND UBIQUITOUS COMPUTING, ACM, 2 PENN PLAZA, SUITE 701 NEW YORK NY 10121-0701 USA, 12 September 2016 (2016-09-12), pages 886-897, XP058279171, DOI: 10.1145/2971648.2971740 ISBN: 978-1-4503-4461-6
- KAREN HOVSEPIAN ET AL: "cStress", PERVASIVE AND UBIQUITOUS COMPUTING, ACM, 2 PENN PLAZA, SUITE 701 NEW YORK NY 10121-0701 USA, 7 September 2015 (2015-09-07), pages 493-504, XP058074083, DOI: 10.1145/2750858.2807526 ISBN: 978-1-4503-3574-4

## Description

### BACKGROUND:

### Field:

Certain embodiments generally relate to systems and methods, as set out in the appended set of claims, for utilizing sensor data from user devices, such as smartphones and smart wearable devices, to infer or estimate the stress valence of users of such user devices.

### Description of the Related Art:

In wireless telecommunications systems, such as Long Term Evolution or 5^{th} generation (5G) systems, smartphones and wearable devices (e.g., smart watches or activity trackers) may be generally referred to as user equipment (UE). In LTE or 5G systems, a UE may communicate with base stations (which may also be referred to as Node Bs or evolved Node Bs (eNBs)) to allow for connectivity between the user equipment (UE) and the core network.

Nowadays, user equipment (UE), such as smartphones and wearable devices, may incorporate sensors to monitor a user's fitness activity and physiological data. For example, many UEs include integrated pedometers for counting a user's steps and accelerometers or other sensors for calculating mileage, overall physical activity, calorie expenditure, and to monitor heart rate and quality of sleep, etc.

Outside of generally tracking activity level, an important application for these sensors is for detecting and managing stress. Stress is clearly a significant problem in modern-day medicine, and stress and stress-related symptoms are a major cause of physical and psychological illnesses. Consequently, methods and systems for monitoring and inferring the stress valence in individuals can provide significant benefits to individuals and society as a whole.

The Article 'Finding Significant Stress Episodes in a Discontinuous Time Series of Rapidly Varying Mobile Sensor Data' by H Sarker et al (XP058257306) discloses that management of daily stress can be greatly improved by delivering sensor-triggered just-in-time interventions (JITIs) on mobile devices. The success of such JITIs critically depends on being able to mine the time series of noisy sensor data to find the most opportune moments. The paper proposes a time series pattern mining method to detect significant stress episodes in a time series of discontinuous and rapidly varying stress data.

The Article "Assessing the availability of users to engage in just-in-time intervention in the natural environment" by H Sarker et al discloses wearable wireless sensors for health monitoring are enabling the design and delivery of just-in-time interventions (JITI). It is noted that JITI is critical to the success of JITI is to time its delivery so that the user is available to be engaged.

The Article "CrossCheck" by Wang et al discloses a first step towards passive monitoring of mental health indicators in patients with schizophrenia and paves the way towards relapse prediction and early intervention. In this paper, initial results are presented where passive smartphone sensor data is collected from 21 outpatients with schizophrenia recently discharged from hospital over a period ranging from 2-8.5 months. The results indicate that there are associations between automatically tracked behavioral features related to sleep, mobility, conversations, smart-phone usage and self-reported indicators of mental health in schizophrenia.

The Article "cStress" by K Hobsepian et al. discloses a stress model (called *cStress*). The model was trained using data collected from a rigorous lab study with 21 participants and validated on two independently collected data sets.

### SUMMARY:

The scope of protection sought for various embodiments of the invention is set out by the independent claims. The embodiments and features, if any, described in the specification that do not fall under the scope of the independent claims are to be interpreted as examples useful for understanding various embodiments of the invention.

### BRIEF DESCRIPTION OF THE DRAWINGS:

For proper understanding of the present disclosure, reference should be made to the accompanying drawings, wherein:
Fig. 1 illustrates a system according to one embodiment of the present disclosure;
Fig. 2a illustrates an apparatus according to one embodiment;
Fig. 2b illustrates an apparatus according to another embodiment;
Fig. 3 illustrates an example graph depicting a process of performing dynamic temporal backtracking to infer or estimate stress valence, according to certain embodiments;
Fig. 4 illustrates an example of a graph depicting a user's activities, according to an embodiment; and
Fig. 5 illustrates a flow diagram of a method, according to one embodiment.

### DETAILED DESCRIPTION:

It will be readily understood that the components of the present disclosure, as generally described and illustrated in the figures herein, may be arranged and designed in a wide variety of different configurations. Thus, the following detailed description of the optional embodiments of systems, methods, apparatuses, and computer program products for inferring the stress valence of an individual from sensor data, is not intended to limit the scope of the present disclosure but is representative of selected embodiments of the present disclosure.

The features, structures, or characteristics of the present disclosure described throughout this specification may be combined in any suitable manner in one or more embodiments. For example, the usage of the phrases "certain embodiments," "some embodiments," or other similar language, throughout this specification refers to the fact that a particular feature, structure, or characteristic described in connection with the embodiment may be included in at least one embodiment of the present disclosure. Thus, appearances of the phrases "in certain embodiments," "in some embodiments," "in other embodiments," or other similar language, throughout this specification do not necessarily all refer to the same group of embodiments, and the described features, structures, or characteristics may be combined in any suitable manner in one or more embodiments.

Additionally, if desired, the different functions discussed below may be performed in a different order and/or concurrently with each other. Furthermore, if desired, one or more of the described functions may be optional or may be combined. As such, the following description should be considered as merely illustrative of the principles, teachings and embodiments of this present disclosure, and not in limitation thereof.

As introduced above, one embodiment is directed to a method for estimating or inferring the stress valence of an individual from at least one sensor data. Stress generally refers to an individual's response to an event, environmental condition or external stimulus. Negative stress may refer to a state of mental or emotional strain or tension resulting from adverse or very demanding events or conditions. Following a stressful event, the body's way of responding to the stress may be by sympathetic nervous system activation which may result, for example, in a fight-or-flight response. Therefore, in humans, stress usually describes a negative condition that can affect a person's mental and physical well-being. However, some events or conditions may actually elicit a positive reaction or response from the body. Accordingly, physiological stress may have beneficial effects where it is termed eustress and may have negative effects where it is termed distress.

Valence, as used in the context of emotions or stress, refers to the intrinsic attractiveness (positive valence) or averseness (negative valence) of an event, object, or situation. The term can also be used to characterize specific emotions. For instance, the emotions usually referred to as "negative", such as anger and fear, have "negative valence". While positive emotions, such as joy or pleasure, have "positive valence". Thus, positively valenced emotions are evoked by positively valenced events, objects, or situations. As used herein, stress valence can refer to negative stress that may be the result of adverse conditions or to positive stress that may be the result of favorable conditions.

User equipment including, but not limited to, smartphones and wearable devices may include various types of sensors to obtain contextual data and physiological data from the user. The types of sensors that may incorporated into UEs can include, for example, pedometers, inertial measurement units (IMUs), accelerometers, gyroscopes, magnetometer, barometers, optical sensors, electrodes, etc. Contextual data that may be obtained from such sensors may include, for example, geographic location, time of day, altitude, external temperature, other environmental elements, or any combination thereof. Physiological data that may be obtained from such sensors may include, for example, the user's physical or biological characteristics including heart rate, pulse, core body temperature, respiration rate, blood oxygenation level, blood pressure, skin temperature, cardiac rhythm, or any combination thereof. According to one embodiment, the user equipment (or sensors therein) may continuously measure or obtain the contextual and physiological data from the user. In other embodiments, the user equipment (or sensors) may discontinuously measure or obtain the contextual and physiological data.

An additional approach for obtaining information or data from a user is by sending one or more questionnaires, such as ecological momentary assessments (EMAs). EMAs are a psychological science technique that encourages individuals to record emotional states, moods, thoughts, and actions at a particular time. In an embodiment, one or more EMA(s) may be transmitted to user equipment for display on a screen of the user equipment, for example. According to certain embodiments, responses provided by the user to the EMA(s) may be converted into machine readable and analyzable formats.

However, EMAs may impose a high burden on a user and the level or frequency of response to EMAs from users may decrease over time. This is because sending EMAs to users for feedback can be highly interruptive and onerous to the users. Certain embodiments are able to reduce the amount of interruption experienced by users thereby resulting in better user acceptance and feedback. For example, an embodiment is configured to: filter physiological arousal unrelated to stress (e.g., physical activity), trigger EMAs only when the arousal is significant (rather than triggering EMAs randomly or periodically), consider habit information to determine stressors, and to transmit EMAs at appropriate times (e.g., in case of negative valence). As a result, certain embodiments significantly reduce false triggers for sending EMAs, and increase user acceptance and feedback.

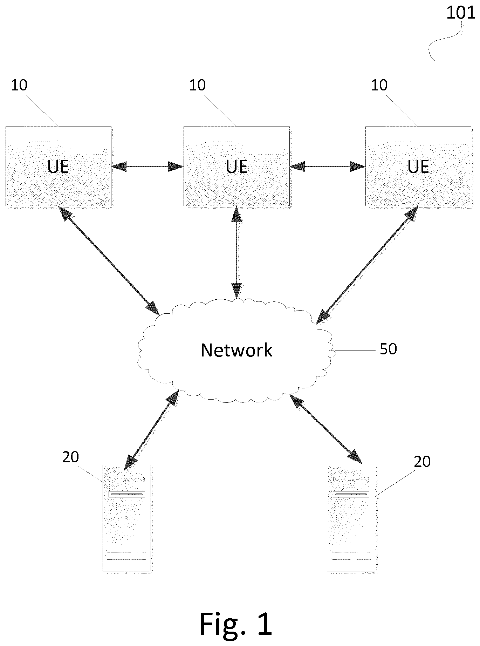

Fig. 1 illustrates a system 101 according to one embodiment of the present disclosure. In the example of Fig. 1, system 101 may include a network 50. The network 50 may be a radio access network, such as LTE or 5G, a local area network (LAN), a wide area network (WAN) such as the Internet, or wireless LAN (WLAN), for example. As illustrated in the example of Fig. 1, one or more UE(s) 10 may be configured to communicate with the network 50 via wireless or wired connections. In an addition, in an embodiment, the UE(s) 10 may be configured to communicate directly with each other via wireless or wired connections. Examples of UE(s) 10 may include, but is not limited to, smartphones, wearable devices, tablets, laptop computers, desktop computers, or other mobile or stationary device. In an embodiment, system 101 may also include one or more hosts or servers 20 connected to the network 50 through wireless or wired connections. According to one embodiment, servers 20 may be implemented in or function as base stations or eNBs. In other embodiments, servers 20 may include web servers, mail servers, application servers, etc. According to certain embodiments, servers 20 may be standalone servers, networked servers, or an array of servers.

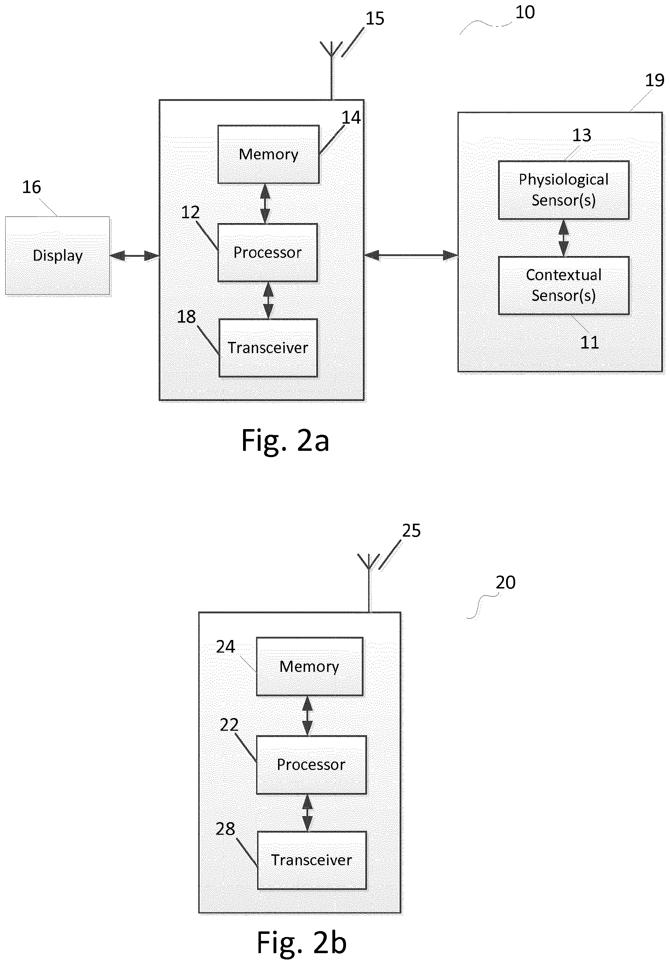

Fig. 2a illustrates an example of a UE or apparatus 10 according to an embodiment. In an embodiment, apparatus 10 may be a node or element in a communications network or associated with such a network, such as a mobile device, smartphone, wearable device, tablet computer, laptop computer, Internet of Things (IoT) device, or other mobile or stationary device. As described herein, apparatus 10 may alternatively be referred to as, for example, a UE, mobile station, mobile equipment, mobile unit, mobile device, user device, subscriber station, wireless terminal, tablet, smartphone, wearable device, IoT device, or the like. As one example, Apparatus 10 may be implemented in, for instance, a wireless handheld device, a wireless wearable device, a wireless plug-in accessory, or any combination thereof. In one example, apparatus 10 may correspond to UE(s) 10 depicted in Fig. 1 discussed above. It should be noted that one of ordinary skill in the art would understand that apparatus 10 may include components or features not shown in Fig. 2a.

As illustrated in Fig. 2a, apparatus 10 may include a processor 12 for processing information and executing instructions or operations. Processor 12 may be any type of general or specific purpose processor. While a single processor 12 is shown in Fig. 2a, multiple processors may be utilized according to other embodiments. In fact, processor 12 may include one or more of general-purpose computers, special purpose computers, microprocessors, digital signal processors (DSPs), field-programmable gate arrays (FPGAs), application-specific integrated circuits (ASICs), and processors based on a multi-core processor architecture, as examples. In some cases, the processor 12 may be remote from the apparatus 10, such as disposed within a server like server 20 of Fig. 1.

Processor 12 may perform functions associated with the operation of apparatus 10 which may include, for example, precoding of antenna gain/phase parameters, encoding and decoding of individual bits forming a communication message, formatting of information, and overall control of the apparatus 10, including processes related to management of communication resources.

Apparatus 10 may further include or be coupled to a memory 14 (internal or external), which may be coupled to processor 12, for storing information and instructions that may be executed by processor 12. Memory 14 may be one or more memories and of any type suitable to the local application environment, and may be implemented using any suitable volatile or nonvolatile data storage technology such as a semiconductor-based memory device, a magnetic memory device and system, an optical memory device and system, fixed memory, and removable memory. For example, memory 14 can be comprised of any combination of random access memory (RAM), read only memory (ROM), static storage such as a magnetic or optical disk, hard disk drive (HDD), or any other type of non-transitory machine or computer readable media. The instructions stored in memory 14 may include program instructions or computer program code that, when executed by processor 12, enable the apparatus 10 to perform tasks as described herein.

In some embodiments, apparatus 10 may also include or be coupled to one or more antennas 15 for transmitting and receiving signals and/or data to and from apparatus 10. Apparatus 10 may further include or be coupled to a transceiver 18 configured to transmit and receive information. The transceiver 18 may include, for example, a plurality of radio interfaces that may be coupled to the antenna(s) 15. The radio interfaces may correspond to a plurality of radio access technologies including one or more of LTE, 5G, WLAN, Bluetooth, near field communication (NFC), radio frequency identifier (RFID), ultrawideband (UWB), and the like. The radio interface may include components, such as filters, converters (for example, digital-to-analog converters and the like), mappers, a Fast Fourier Transform (FFT) module, and the like, to generate symbols for a transmission via one or more downlinks and to receive symbols (for example, via an uplink). As such, transceiver 18 may be configured to modulate information on to a carrier waveform for transmission by the antenna(s) 15 and demodulate information received via the antenna(s) 15 for further processing by other elements of apparatus 10. In other embodiments, transceiver 18 may be capable of transmitting and receiving signals or data directly.

Apparatus 10 may further include a display 16, such as a touchscreen, which may include a user interface, such as a graphical user interface. In certain embodiments, display 16 may be configured to display information or data to a user of apparatus 10. Display 16 may also be configured to receive input from a user of apparatus 10 via a touchscreen, for example.

In an embodiment, memory 14 may store software modules that provide functionality when executed by processor 12. The modules may include, for example, an operating system that provides operating system functionality for apparatus 10. The memory may also store one or more functional modules, such as an application or program, to provide additional functionality for apparatus 10. The components of apparatus 10 may be implemented in hardware, or as any suitable combination of hardware and software.

According to an embodiment, apparatus 10 may include or be connected to one or more sensors 19. Sensors 19 may include physiological sensor(s) 13 and/or context sensor(s) 11. Sensors 19 may include one or more of pedometers, inertial measurement units (IMUs), accelerometers, gyroscopes, magnetometer, barometers, optical sensors, electrodes, etc. Contextual data that may be obtained from context sensor(s) 11 may include, for example, geographic location, time of day, altitude, external temperature or any combination thereof. Physiological data that may be obtained from physiological sensor(s) 13 may include, for example, the user's physical or biological characteristics including heart rate, pulse, core body temperature, respiration rate, blood oxygenation level, blood pressure, skin temperature, cardiac rhythm, or any combination thereof.

In one embodiment, apparatus 10 may be a UE, mobile station, mobile equipment, mobile unit, mobile device, user device, subscriber station, wireless terminal, tablet, smartphone, wearable device, IoT device, for example. According to certain embodiments, apparatus 10 may be controlled by memory 14 and processor 12 to perform the functions associated with embodiments described herein. For example, in an embodiment, apparatus 10 may be controlled by memory 14 and processor 12 to measure and/or collect a first set of data from at least one sensor 19. The first set of data may include physiological data and/or contextual data, for example. According to one embodiment, apparatus 10 (or sensor(s) 19) may be controlled by memory 14 and processor 12 to continuously measure or obtain the contextual and/or physiological data from the user. In other embodiments, apparatus 10 (or sensor(s) 19) may be controlled to discontinuously measure or obtain the contextual and/or physiological data.

Apparatus 10 is further controlled by memory 14 and processor 12 to collect a second set of data that includes one or more partial or complete ecological momentary assessment (EMA) response(s) from a user of apparatus 10. In one embodiment, ecological momentary assessments (EMAs) may refer to a questionnaire that requests certain information from individuals, such as their emotional states, moods, thoughts, and actions at a particular time. Accordingly, an EMA response contains may contain information on the stress of a user including, for example, the stress valence (i.e., positive or negative stress) at the time of the EMA response. In an embodiment, apparatus 10 may be controlled by memory 14 and processor 12 to provide or display one or more EMA(s) on a screen such as the display 16 of the apparatus 10, for example, at appropriate times. According to certain embodiments, responses provided by the user to the EMA(s) may be converted into machine readable and analyzable formats.

According to an embodiment, apparatus 10 may be controlled by memory 14 and processor 12 to store the first set of data and the second set of data in memory 14. In one embodiment, the first set of data may be continuous data that contains information on a level of arousal or stimulation of the user, such as heart rate and heart rate variation data. In an embodiment, information contained in an EMA response from a user may be considered as discontinuous data that contains information on the stress of the user, such as valence (i.e., positive or negative stress reported in the EMA response).

Apparatus 10 is further controlled by memory 14 and processor 12 to establish a set of physiological time-series data for a user of apparatus 10 based on the first set of data and the second set of data. For example, in an embodiment, apparatus 10 may be controlled by memory 14 and processor 12 to establish the set of physiological time-series data by combining, integrating and/or synchronizing the first set of data (e.g., the physiological and contextual data) and the second set of data (e.g., one or more EMA responses) to create the set of physiological time-series data for the user. According to an embodiment, the combining, integrating and/or synchronizing of the first set of data and the second set of data may include establishing consistency among the sets of data, which may be collected from different sources, and the continuous harmonization of the sets of data over time. The physiological time-series data may be a representation of the user's physical and/or emotional state at a given time based on the contextual data, physiological data, and/or EMA response data collected. For example, in one embodiment, the physiological time-series data may include a representation of the user's physiological stimulation (e.g., elevated heart rate) combined with contextual data (e.g., time or location).

In another embodiment, apparatus 10 may be controlled by memory 14 and processor 12 to transmit the first set of data (e.g., the physiological and contextual data) and the second set of data (e.g., one or more EMA responses) to a node, host or server of a network. According to this embodiment, the network node may be configured to establish the set of physiological time-series data for a user of apparatus 10 based on the received first set of data and second set of data. For instance, in this embodiment, the network node may establish the set of physiological time-series data by combining, integrating and/or synchronizing the first set of data and the second set of data to create the set of physiological time-series data for the user. As discussed in the foregoing, the combining, integrating and/or synchronizing of the first set of data and the second set of data may include establishing consistency among the sets of data, which may be collected from different sources, and the continuous harmonization of the sets of data over time.

Apparatus 10 is controlled by memory 14 and processor 12 to identify habit information related to regularly occurring habits of the user based on the first set of data (e.g., physiological and contextual data). In an embodiment, habit information refers to information representative of regularly occurring habits or routines that make up a user's day. In other words, as one example, habits refer to a repeated series of activities that an individual performs on a regular day or basis. For example, habits for an average individual may include sleeping, eating meals, commuting, working, exercising, etc. According to one embodiment, apparatus 10 may be controlled by memory 14 and processor 12 to place a habit anchor within the physiological time-series data to identify periods of time during which the regularly occurring habits are happening.

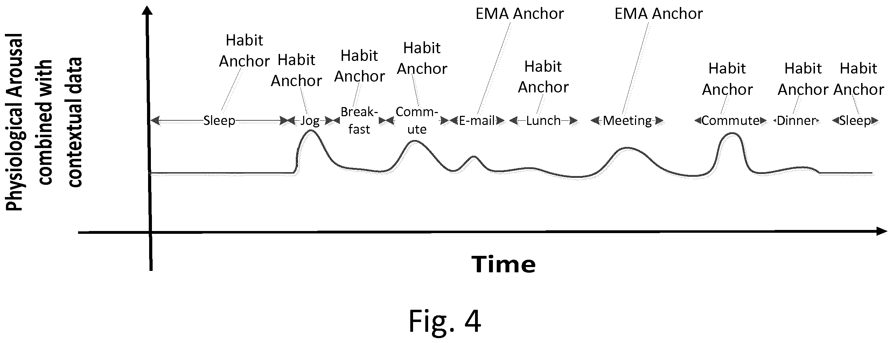

Fig. 4 is an example of a graph depicting a user's activities during the twenty four hours of a day. In the example of Fig. 4, the x-axis depicts the time of day and the y-axis depicts the physiological arousal of the user. Fig. 4 shows several daily habits which may be repeated over workdays at similar times. Fig. 4 also shows the physiological change in the user, their time, duration, and when a physiological change may coincide with the habits, throughout the day. Fig. 4 further illustrates some other events, such as checking e-mail or attending meetings, which may occur irregularly or sporadically throughout a day. These irregular events may also cause physiological changes, as shown in Fig. 4. In the example of Fig. 4, habit anchors may be placed at periods where habits usually occur, such as during times of sleep, jogging, eating breakfast, commuting to work, eating lunch, commuting back home, and eating dinner, for instance. In an embodiment, EMA anchors may be placed during the times of other events (e.g., meetings or checking e-mail). Thus, stress valence during habits may be captured via habit anchor, and during other events via EMA anchor. Once the habit information is captured, apparatus 10 can determine whether a habit creates positive or negative stress, or if it has a neutral effect on the user. It is noted that Fig. 4 is merely one example and many other possibilities or examples are applicable according to certain embodiments of the present disclosure.

Apparatus 10 may be controlled by memory 14 and processor 12 to determine or detect, based on at least the first set of data (e.g., physiological and contextual data) and the habit information, a potential trigger point for sending an EMA to the user. The EMA is used, in part, to ascertain the stress valence of the user at the time of the potential trigger point. According to one embodiment, the potential trigger point may be a point in time where the first set of data shows a significant change in level that is above or below a pre-defined threshold. As one example, if the physiological sensor 13 detects that the user's heart rate has moved above 100 beats per minute (BPM) and the habit information does not reflect a reason for the elevated heart rate, then apparatus 10 may be controlled to determine that this is a potential trigger point for sending the EMA to the user. However, if the physiological sensor 13 detects that the user's heart rate has moved above 100 beats per minute (BPM) but the habit information indicates that the user is likely exercising (i.e., this is the time of day the user normally exercises or the user location is at the gym), then apparatus 10 may determine that this is not a potential trigger point for sending the EMA to the user. In other words, apparatus 10 uses the habit information to disregard physiological stimulation or arousal unrelated to psychological stress. It is noted that heart rate is merely one example of physiological or contextual data that may cause apparatus 10 to detect a potential trigger point, as any physiological or contextual data can similarly be used according to other embodiments.

At the time of the potential trigger point, apparatus 10 is controlled by memory 14 and processor 12 to compute or calculate an estimate of the stress valence value associated with the EMA for which an EMA response was at least partially not received (e.g., either because the EMA was not sent or because the user did not provide a response to the EMA). The stress valence value estimate is calculated based on at least one of the physiological time-series data, at least one previously collected partial or complete EMA response, the first set of data, the habit information, or any combination thereof.

The computing or calculating of the estimate of the stress valence value includes apparatus 10 being controlled by memory 14 and processor 12 to perform dynamic temporal backtracking to identify past trends in the physiological time-series data of the user. Apparatus 10 is controlled by memory 14 and processor 12 to iteratively apply an increasing retrospective time window beginning from the time of the potential trigger point until a previously captured EMA response is found representing a change in physiological data, contextual data, or any combination thereof, that is similar to the significant change in the first set of data that caused the detection of the potential trigger point. In this embodiment, when the previously captured EMA response is found that shows the similar change, apparatus 10 may be controlled by memory 14 and processor 12 to use the stress valence determined from the previously captured EMA response as the estimated stress valence value for the user at the potential trigger point.

As noted above, the detection of a potential trigger point does not necessarily mean that an EMA will be sent to the user. However, in one embodiment, upon detection of the potential trigger point, apparatus 10 may be controlled by memory 14 and processor 12 to send the EMA to the display 16 so that the user can provide an EMA response. In an embodiment, apparatus 10 may then be controlled by memory 14 and processor 12 to receive the EMA response from the user and to use the EMA response to determine the stress valence of the user. In another embodiment, a response to the EMA may not be provided by the user. According to an embodiment, apparatus 10 may then be controlled by memory 14 and processor 12 to calculate the estimate of the stress valence value associated with the EMA when a response to the subsequent EMA is not received.

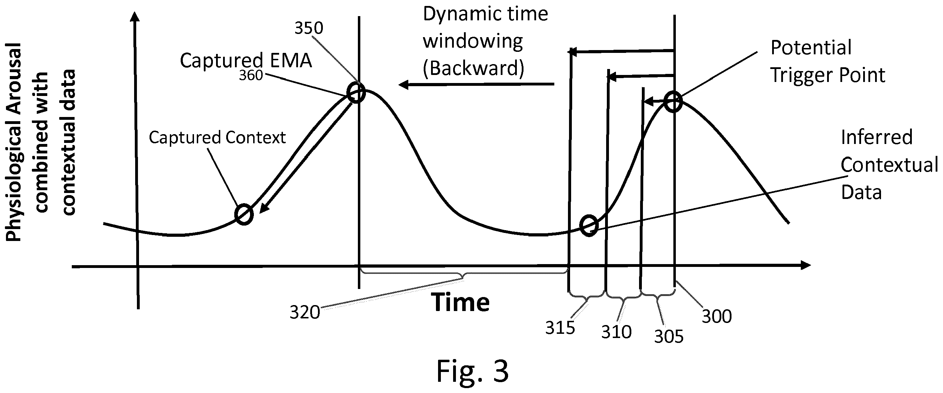

Fig. 3 illustrates an example graph depicting how apparatus 10 may perform dynamic temporal backtracking to infer or estimate stress valence, according to certain embodiments. The example of Fig. 3 illustrates how stress valence may be determined using retrospective time window(s) to capture the most similar valence response (i.e., EMA response) in terms of physiological arousal/stimulation, context(s) (e.g., time of day), while accounting for habit information (e.g., exercising, commuting, etc.).

As illustrated in the example of Fig. 3, the potential trigger point is detected at 300 because of a spike or change in the level of the first set of data (i.e., physiological and context data) that exceeds a predetermined threshold. For example, in an embodiment, the potential trigger point may be an initiation event defined as a period of protracted arousal as measured by the first set of data (e.g., physiological and contextual data). As discussed above, the first set of data may show changes in arousal throughout a day that are unrelated to stress (e.g., from exercising); however, in an embodiment, the potential trigger point 300 is detected based on a protracted period of arousal that exceeds a pre-determined threshold. The duration of the protracted time period may vary for each individual, for example based on level of health or any pre-existing health conditions.

Thus, according to an embodiment, beginning at the potential trigger point 300, apparatus 10 may be controlled to track backwards in time to identify the start of the event that may have caused the change in the level of the first set of data. For example, in one embodiment, apparatus 10 may be controlled to iteratively apply retrospective time windows 305, 310, 315, 320 of increasing duration to, in effect, look back over the preceding time with progressively longer windows and averaging arousal to confirm that it is unchanging over a time period. The duration of the time windows may vary empirically based on the amount and type of data collected or measured. This process of retrospective averaging may stop when the earliest time stamp coincides with the completion time of an EMA response addressing stress valence of the user. This EMA response could then be integrated with the first set of data to determine if the stress observed is positive or negative.

In the example of Fig. 3, apparatus 10 may be controlled to iteratively apply the retrospective time windows until time 350 where a previous EMA response 360 was captured when there was a spike or change in the set of first data that is similar to the change detected at the potential trigger point 300. In one embodiment, apparatus 10 may be controlled to use the stress valence determined from the previously captured EMA response 360 as the estimated stress valence value for the user at the potential trigger point 300.

In one embodiment, apparatus 10 may be controlled by memory 14 and processor 12 to provide a user interface for a user to review auto-generated triggers with associated meaningful contexts in offline or online modes, and to provide users with the opportunity to provide feedback on the accuracy of the triggers. Certain embodiments are then able to adapt the user feedback and improve its accuracy in determining stress valance and triggering EMA(s). Moreover, in an embodiment, apparatus 10 may be controlled by memory 14 and processor 12 to accept priority rules defined by a user to avoid interruptions whenever they want.

Fig. 2b illustrates an example of an apparatus 20 according to another embodiment. In an embodiment, apparatus 20 a node, host, or server in a communications network or serving such a network. For example, apparatus 20 may be a base station, a node B, an evolved node B, 5G node B or access point, next generation node B (NG-NB), WLAN access point, mobility management entity (MME), or subscription server associated with a radio access network, such as a GSM network, LTE network or 5G radio access technology. For instance, in an embodiment, apparatus 20 may correspond to network node 20 illustrated in Fig. 1 discussed above. It should be noted that one of ordinary skill in the art would understand that apparatus 20 may include components or features not shown in Fig. 2b.

As illustrated in Fig. 2b, apparatus 20 may include or be coupled to a processor 22 for processing information and executing instructions or operations. Processor 22 may be any type of general or specific purpose processor. While a single processor 22 is shown in Fig. 2b, multiple processors may be utilized according to other embodiments. In fact, processor 22 may include one or more of general-purpose computers, special purpose computers, microprocessors, digital signal processors (DSPs), field-programmable gate arrays (FPGAs), application-specific integrated circuits (ASICs), and processors based on a multi-core processor architecture, as examples.

Processor 22 may perform functions associated with the operation of apparatus 20 including, without limitation, precoding of antenna gain/phase parameters, encoding and decoding of individual bits forming a communication message, formatting of information, and overall control of the apparatus 20, including processes related to management of communication resources.

Apparatus 20 may further include or be coupled to a memory 24 (internal or external), which may be coupled to processor 22, for storing information and instructions that may be executed by processor 22. Memory 24 may be one or more memories and of any type suitable to the local application environment, and may be implemented using any suitable volatile or nonvolatile data storage technology such as a semiconductor-based memory device, a magnetic memory device and system, an optical memory device and system, fixed memory, and removable memory. For example, memory 24 can be comprised of any combination of random access memory (RAM), read only memory (ROM), static storage such as a magnetic or optical disk, or any other type of non-transitory machine or computer readable media. The instructions stored in memory 24 may include program instructions or computer program code that, when executed by processor 22, enable the apparatus 20 to perform tasks as described herein.

In some embodiments, apparatus 20 may also include or be coupled to one or more antennas 25 for receiving a downlink signal and for transmitting via an uplink from apparatus 20. Apparatus 20 may further include a transceiver 28 configured to transmit and receive information. The transceiver 28 may also include a radio interface (e.g., a modem) coupled to the antenna 25. The radio interface may correspond to a plurality of radio access technologies including one or more of GSM, LTE, LTE-A, 5G, WLAN, IoT, Bluetooth, NFC, RFID, UWB, and the like. The radio interface may include other components, such as filters, converters (for example, digital-to-analog converters and the like), symbol demappers, signal shaping components, an Inverse Fast Fourier Transform (IFFT) module, and the like, to process symbols, such as OFDMA symbols, carried by a downlink or an uplink.

For instance, transceiver 28 may be configured to modulate information on to a carrier waveform for transmission by the antenna(s) 25 and demodulate information received via the antenna(s) 25 for further processing by other elements of apparatus 20. In other embodiments, transceiver 28 may be capable of transmitting and receiving signals or data directly. Apparatus 20 may further include a user interface, such as a graphical user interface or touchscreen.

In an embodiment, memory 24 stores software modules that provide functionality when executed by processor 22. The modules may include, for example, an operating system that provides operating system functionality for apparatus 20. The memory may also store one or more functional modules, such as an application or program, to provide additional functionality for apparatus 20. The components of apparatus 20 may be implemented in hardware, or as any suitable combination of hardware and software.

According to one embodiment, apparatus 20 may be a network node or server, such as a base station, node B, eNB, 5G node B or access point, or next generation node B (NG-NB), for example. Apparatus 20 is controlled by memory 24 and processor 22 to perform the functions associated with embodiments described herein. Apparatus 20 is controlled by memory 24 and processor 22 to receive and/or collect a first set of data from one or more sensors of one or more user devices. The first set of data includes physiological data and/or contextual Apparatus 20 is controlled by memory 24 and processor 22 to continuously receive or obtain the contextual and/or physiological data from the sensor(s) of the user device(s). Thus, in one embodiment, the first set of data may be continuous data that contains information on a level of arousal or stimulation of the user, such as heart rate and heart rate variation data. In other embodiments, apparatus 20 may be controlled to discontinuously receive or obtain the contextual and/or physiological data.

Apparatus 20 is controlled by memory 24 and processor 22 to receive and/or collect a second set of data that includes one or more partial or complete ecological momentary assessment (EMA) response(s) from a user of the user device(s). As discussed above, in one embodiment, ecological momentary assessments (EMAs) may refer to a questionnaire that requests certain information from individuals, such as their emotional states, moods, thoughts, and actions at a particular time. As such, an EMA response contains information on the stress of a user including the stress valence (i.e., positive or negative stress) at the time of the EMA response.

In an embodiment, apparatus 20 may be controlled by memory 24 and processor 22 to transmit one or more EMA(s) to the user device(s) for display on a screen of the user device(s), for example, at appropriate times. According to certain embodiments, apparatus 20 may be controlled by memory 24 and processor 22 to receive responses provided by the user to the EMA(s) and to convert the responses into machine readable and analyzable formats. In an embodiment, information contained in an EMA response from a user may be considered as discontinuous data that contains information on the stress of the user, such as valence (i.e., positive or negative stress reported in the EMA response). According to an embodiment, apparatus 20 may be further controlled by memory 24 and processor 22 to store the first set of data and the second set of data in memory 24. In other embodiments, the user device may be configured to itself trigger display of one or more EMA(s), for example, on the screen of the user device, without involvement of apparatus 20.

Apparatus 20 is further controlled by memory 24 and processor 22 to establish a set of physiological time-series data for a user of the user device, for example, based on the first set of data and the second set of data. For example, in an embodiment, apparatus 20 may be controlled by memory 24 and processor 22 to establish the set of physiological time-series data by combining, integrating and/or synchronizing the first set of data (e.g., the physiological and contextual data) and the second set of data (e.g., one or more EMA responses) to create the set of physiological time-series data for the user. According to an embodiment, the combining, integrating and/or synchronizing of the first set of data and the second set of data may include establishing consistency among the sets of data, which may be collected from different sources, and the continuous harmonization of the sets of data over time.

For instance, in an embodiment, the first set of data may need to be synchronized together since the first set of data may include measurements taken from different sensors at different times. As one example, the first set of data may include motion measured by an accelerometer and heart rate measured by a physiological sensor. Accordingly, apparatus 20 may be controlled to synchronize and integrate the motion measurement with the heart rate measurement. In addition, the first set of data may need to be synchronized with the second set of data (e.g., an EMA response), for example based on collection times. In other embodiments, the processing, combining, integrating and/or synchronizing of the first set of data (e.g., the physiological and contextual data) and the second set of data (e.g., EMA response(s)) may be performed either in the user device (e.g., smartphone) or the apparatus 20 connected in the network, or any combination thereof. Similarly, the EMA may be triggered either from the apparatus 20 to the user device, or by the user device alone.

The physiological time-series data is a representation of the user's physical and/or emotional state at a given time based on the contextual data, physiological data, and/or EMA response data collected. For example, in one embodiment, the physiological time-series data may include a representation of the user's physiological stimulation (e.g., elevated heart rate) combined with contextual data (e.g., time or location).

Apparatus 20 is further controlled by memory 24 and processor 22 to identify habit information related to regularly occurring habits of the user based on the first set of data (e.g., physiological and contextual data). As discussed above, in one example, habits may refer to a repeated series of activities that an individual performs on a regular day or basis, such as sleeping, eating meals, commuting, working, exercising, etc. According to one embodiment, apparatus 20 may be further controlled by memory 24 and processor 22 to place a habit anchor within the physiological time-series data to identify periods of time during which the regularly occurring habits are happening.

As described above in connection with Fig. 4, in an embodiment, habit anchors may be placed at periods where habits usually occur, such as during times of sleep, jogging, eating breakfast, commuting to work, eating lunch, commuting back home, and eating dinner, for instance. According an embodiment, EMA anchors may be placed during the times of other events (e.g., meetings or checking e-mail). Thus, stress valence during habits may be captured via habit anchor, and during other events via EMA anchor. Once the habit information is captured, apparatus 20 may be controlled to determine whether a habit creates positive or negative stress, or if it has a neutral effect on the user.

Apparatus is further controlled by memory 24 and processor 22 to determine or detect, based on at least the first set of data (e.g., physiological and contextual data) and the habit information, a potential trigger point for sending an EMA to the user. In an embodiment, the EMA may be used, in part, to ascertain the stress valence of the user at the time of the potential trigger point. According to one embodiment, the potential trigger point may be a point in time where the first set of data shows a significant change in level that is above or below a pre-defined threshold. Apparatus 20 uses the habit information to disregard physiological stimulation or arousal unrelated to stress.

At the time of the potential trigger point, apparatus 20 is further controlled by memory 24 and processor 22 to compute or calculate an estimate of the stress valence value associated with the EMA for which an EMA response was at least partially not received (e.g., either because the EMA was not sent or because the user did not provide a response to the EMA). The stress valence value estimate may be calculated based on at least one of the physiological time-series data, at least one previously collected partial or complete EMA response, the first set of data, or the habit information.

The computing or calculating of the estimate of the stress valence value includes apparatus 20 being controlled by memory 24 and processor 22 to perform dynamic temporal backtracking to identify past trends in the physiological time-series data of the user. As discussed above in connection with Fig. 3, apparatus 20 may be further controlled by memory 24 and processor 22 to iteratively apply an increasing retrospective time window beginning from the time of the potential trigger point until a previously captured EMA response is found representing a change in physiological data, contextual data, or any combination thereof, that is similar to the significant change in the first set of data that caused the detection of the potential trigger point. When the previously captured EMA response is found that shows the similar change, apparatus 20 is further controlled by memory 24 and processor 22 to use the stress valence determined from the previously captured EMA response as the estimated stress valence value for the user at the potential trigger point.

As noted above, the detection of a potential trigger point does not necessarily result in an EMA being sent to the user. However, in one embodiment, upon detection of the potential trigger point, apparatus 20 may be further controlled by memory 24 and processor 22 to send the EMA to the user device(s) so that the user can provide an EMA response. In an embodiment, apparatus 20 may be further controlled by memory 24 and processor 22 to receive the EMA response from the user and to use the EMA response to determine the stress valence of the user. In another embodiment, a response to the EMA may not be provided by the user. According to an embodiment, apparatus 10 may then be controlled by memory 14 and processor 12 to calculate the estimate of the stress valence value associated with the EMA when a response to the subsequent EMA is not received.

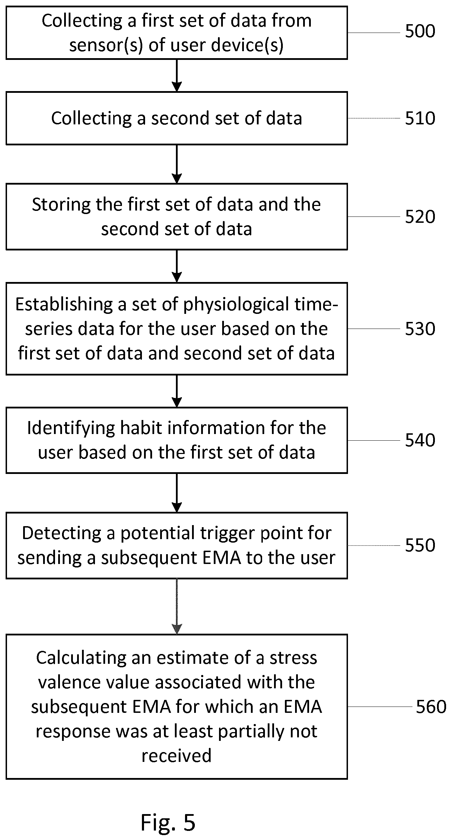

Fig. 5 illustrates an example flow diagram of a method, according to one embodiment. In an embodiment, the method of Fig. 5 may be performed by a user device, such as a UE, smartphone, wearable device, or by a network node, such as a server, base station, node B, eNB, or by any combination thereof.

As illustrated in Fig. 5, the method includes, at 500, measuring and/or collecting a first set of data from one or more sensors of one or more user devices. The first set of data includes physiological data and/or contextual data, for example. According to one embodiment, the collecting 500 may include continuously measuring or obtaining the contextual and/or physiological data from the user device(s). In other embodiments, the collecting 500 may include discontinuously measuring or obtaining the contextual and/or physiological data.

The method further includes, at 510, measuring and/or collecting a second set of data that includes one or more partial or complete ecological momentary assessment (EMA) response(s) from a user of the user device(s). An EMA response contains information on the stress of a user including the stress valence (i.e., positive or negative stress) at the time of the EMA response. In an embodiment, the method may include providing or displaying one or more EMA(s) on a screen of the user device(s), for example, at appropriate times. According to certain embodiments, the method may include receiving response(s) provided by the user to the EMA(s) and converting the EMA responses into machine readable and analyzable formats.

According to an embodiment, the method may also include, at 520, storing the first set of data and the second set of data in memory. In one embodiment, the first set of data may be continuous data that contains information on a level of arousal or stimulation of the user, such as heart rate and heart rate variation data. In an embodiment, information contained in an EMA response from a user may be considered as discontinuous data that contains information on the stress of the user, such as valence (i.e., positive or negative stress reported in the EMA response).

The method then includes, at 530, establishing a set of physiological time-series data for the user based on the first set of data (e.g., the physiological and contextual data) and the second set of data (e.g., one or more EMA responses). For example, in an embodiment, the establishing of the set of physiological time-series data may include combining, integrating and/or synchronizing the first set of data and the second set of data to create the set of physiological time-series data for the user. The physiological time-series data may be a representation of the user's physical and/or emotional state at a given time based on the contextual data, physiological data, and/or EMA response data collected. For example, in one embodiment, the physiological time-series data may include a representation of the user's physiological stimulation (e.g., elevated heart rate) combined with contextual data (e.g., time or location).

In another embodiment, the method may include transmitting the first set of data (e.g., the physiological and contextual data) and the second set of data (e.g., one or more EMA responses) to a node, host or server of a network. According to this embodiment, the network node may be configured to combine and synchronize the first set of data and the second set of data to create the set of physiological time-series data for the user.

The method also includes, at 540, identifying habit information related to regularly occurring habits of the user based on the first set of data (e.g., physiological and contextual data). Habit information refers to information representative of regularly occurring habits or routines that an individual performs on a regular day or basis. According to one embodiment, the method may include placing a habit anchor within the physiological time-series data to identify periods of time during which the regularly occurring habits are happening.

The method further includes, at 550, determining or detecting, based on at least the first set of data (e.g., physiological and contextual data) and the habit information, a potential trigger point for sending an EMA to the user. In an embodiment, the EMA is used, in part, to ascertain the stress valence of the user at the time of the potential trigger point. According to one embodiment, the potential trigger point may be a point in time where the first set of data shows a significant change in level that is above or below a pre-defined threshold. The detecting 550 includes using the habit information (e.g., time and duration of regular physical exercises) to disregard physiological stimulation or arousal unrelated to stress.

At the time of the potential trigger point, the method includes, at 560, computing or calculating an estimate of the stress valence value associated with the EMA for which an EMA response was at least partially not received (e.g., either because the EMA was not sent or because the user did not provide a response to the EMA). The calculating 560 includes calculating the stress valence value estimate based on at least one of the physiological time-series data, at least one previously collected partial or complete EMA response, the first set of data, or the habit information.

The calculating 560 of the estimate of the stress valence value includes performing dynamic temporal backtracking to identify past trends in the physiological time-series data of the user. Performing the dynamic temporal backtracking includes iteratively apply an increasing retrospective time window beginning from the time of the potential trigger point until a previously captured EMA response is found representing a change in physiological data, contextual data, or any combination thereof, that is similar to the significant change in the first set of data that caused the detection of the potential trigger point. When the previously captured EMA response is found that shows the similar change, the calculating 560 includes using the stress valence determined from the previously captured EMA response as the estimated stress valence value for the user at the potential trigger point.

As noted above, the detection of a potential trigger point does not necessarily mean that an EMA will be sent to the user. However, in one embodiment, upon detection of the potential trigger point, the method may include sending the EMA to the user device(s) so that the user can provide an EMA response. In this embodiment, the method may include receiving the EMA response from the user and then the calculating 560 may include using the EMA response to determine the stress valence of the user. In another embodiment, a response to the EMA may not be provided by the user. According to an embodiment, the method may include calculating the estimate of the stress valence value associated with the EMA when a response to the subsequent EMA is not received.

In view of the above, embodiments of the present disclosure may provide several advantages, technical improvements and/or technical effects. For example, certain embodiments improve the functioning of user equipment (UEs), such as smart phones and wearable devices, by making these devices more intuitive and allowing the devices to learn daily habits of users in order to provide feedback or output that is less intrusive and more beneficial to users. As such, embodiments of the present disclosure can improve the performance of network nodes including, for example, UEs, mobile devices, smart phones, wearable devices, network servers, base stations, eNBs, etc. Accordingly, the use of embodiments of the present disclosure results in improved functioning of communications networks and their nodes.

In some embodiments, the functionality of any of the methods, processes, signaling diagrams, or flow charts described herein may be implemented by software and/or computer program code or portions of code stored in memory or other computer readable or tangible media, and executed by a processor.

In certain embodiments, an apparatus may be included or be associated with at least one software application, module, unit or entity configured as arithmetic operation(s), or as a program or portions of it (including an added or updated software routine), executed by at least one operation processor. Programs, also called program products or computer programs, including software routines, applets and macros, may be stored in any apparatus-readable data storage medium and may include program instructions to perform particular tasks.

A computer program product may comprise one or more computer-executable components which, when the program is run, are configured to carry out certain embodiments. The one or more computer-executable components may include software code or portions of it. Modifications and configurations required for implementing the functionality of an embodiment may be performed as routine(s), which may be implemented as added or updated software routine(s). In certain embodiments, software routine(s) may be downloaded into an apparatus.

Software or computer program code or portions of code may be in a source code form, object code form, or in some intermediate form, and may be stored in some type of carrier, distribution medium, or computer readable medium, which may be any entity or device capable of carrying the program. Such carriers include a record medium, computer memory, read-only memory, photoelectrical and/or electrical carrier signal, telecommunications signal, and software distribution package, for example. Depending on the processing power needed, the computer program may be executed in a single electronic digital computer or it may be distributed amongst a number of computers. The computer readable medium or computer readable storage medium may be a non-transitory medium.

In other embodiments, the functionality of any method or process described herein may be performed by hardware, for example through the use of an application specific integrated circuit (ASIC), a programmable gate array (PGA), a field programmable gate array (FPGA), or any other combination of hardware and software. In yet another embodiment, the functionality may be implemented as a signal, a non-tangible means that can be carried by an electromagnetic signal downloaded from the Internet or other network.

According to an embodiment, an apparatus, such as a node, device, or a corresponding component, may be configured as a computer or a microprocessor, such as single-chip computer element, or as a chipset, including at least a memory for providing storage capacity used for arithmetic operations and an operation processor for executing the arithmetic operations.

One having ordinary skill in the art will readily understand that the present disclosure as discussed above may be practiced with steps in a different order, and/or with hardware elements in configurations which are different than those which are disclosed. Therefore, although the present disclosure has been described based upon these preferred embodiments, it would be apparent to those of skill in the art that certain modifications, variations, and alternative constructions would be apparent, while remaining within the scope of the present disclosure. In order to determine the metes and bounds of the present disclosure, therefore, reference should be made to the appended claims.

## Claims

1. A computer-implemented method, comprising:
collecting (500) a first set of data from at least one sensor of at least one user device, wherein the first set of data comprises at least one of physiological data or contextual data;
collecting (510) a second set of data comprising at least one partial or complete ecological momentary assessment (EMA) response from a user of the at least one user device;
establishing (530) a set of physiological time-series data for the user based on the first set of data and the second set of data;
identifying (540) habit information related to regularly occurring habits of the user based on the first set of data;
based on at least the first set of data and the habit information, detecting (550) a potential trigger point for sending a subsequent EMA to the user, the subsequent EMA being used to ascertain a stress valence of the user at a time of the potential trigger point, wherein the detecting of the potential trigger point further comprises using the habit information to disregard physiological arousal unrelated to stress; and
at the time of the potential trigger point, calculating (560) an estimate of a stress valence value associated with the subsequent EMA for which an EMA response was at least partially not received, wherein the stress valence value estimate is calculated based on at least one of the physiological time-series data, the at least one previously collected partial or complete EMA response, the first set of data, or the habit information,
wherein the calculating of the estimate of the stress valence value comprises performing backtracking to identify past trends in the physiological time-series data of the user; and
wherein the performing comprises iteratively applying an increasing retrospective time window beginning from the time of the potential trigger point until a previously captured EMA response is found representing a change in physiological data, contextual data, or any combination thereof, that is similar to the significant change in the first set of data at the potential trigger point; and
the calculating further comprises using a stress valence determined from the previously captured EMA response as the estimated stress valence value for the subsequent EMA.

2. The method according to claim 1, wherein the potential trigger point comprises a point in time where the first set of data shows a significant change in level that is above or below a pre-defined threshold.

3. The method according to any one of claims 1-2, the method further comprising placing a habit anchor within the physiological time-series data to identify periods of time during which the regularly occurring habits are happening.

4. The method according to any one claims 1-3, wherein, upon detection of the potential trigger point, the method further comprises sending the subsequent EMA to the at least one user device, and calculating the estimate of the stress valence value associated with the subsequent EMA when a response to the subsequent EMA is not received.

5. The method according to any one of claims 1-4, wherein the contextual data comprises at least one of geographical location, time of day, altitude, external temperature, or any combination thereof.

6. The method according to any one of claims 1-5, wherein the physiological data comprises the user's physical or biological characteristics including at least one of heart rate, pulse, core body temperature, respiration rate, blood oxygenation level, blood pressure, skin temperature, cardiac rhythm, or any combination thereof.

7. An apparatus, comprising:
at least one processor; and
at least one memory including computer program code,
the at least one memory and the computer program code configured, with the at least one processor, to cause the apparatus at least to
collect (500) a first set of data from at least one sensor of at least one user device, wherein the first set of data comprises at least one of physiological data or contextual data;
collect (510) a second set of data comprising at least one partial or complete ecological momentary assessment (EMA) response from a user of the at least one user device;
establish (530) a set of physiological time-series data for the user based on the first set of data and the second set of data;
identify (540) habit information related to regularly occurring habits of the user based on the first set of data;
detect (550), based on at least the first set of data and the habit information, a potential trigger point for sending a subsequent EMA to the user, the subsequent EMA being used to ascertain a stress valence of the user at a time of the potential trigger point, wherein the detecting of the potential trigger point further comprises using the habit information to disregard physiological arousal unrelated to stress;
at the time of the potential trigger point, calculate (560) an estimate of a stress valence value associated with the subsequent EMA for which an EMA response was at least partially not received, wherein the stress valence value estimate is calculated based on at least one of the physiological time-series data, the at least one previously collected partial or complete EMA response, the first set of data, or the habit information; and
perform backtracking to identify past trends in the physiological time-series data of the user in order to estimate the stress valence value associated with the subsequent EMA, wherein the backtracking comprises iteratively applying an increasing retrospective time window beginning from the time of the potential trigger point until a previously captured EMA response is found representing a change in physiological data, contextual data, or any combination thereof, that is similar to the significant change in the first set of data at the potential trigger point, and wherein the stress valence determined from the previously captured EMA response is used as the estimated stress valence value for the subsequent EMA.

8. The apparatus according to claim 7, wherein the potential trigger point comprises a point in time where the first set of data shows a significant change in level that is above or below a pre-defined threshold.

9. The apparatus according to any one of claims 7 and 8, wherein the at least one memory and the computer program code are further configured, with the at least one processor, to cause the apparatus at least to place a habit anchor within the physiological time-series data to identify periods of time during which the regularly occurring habits are happening.

## Patentansprüche

1. Computerimplementiertes Verfahren, das Folgendes umfasst:
Sammeln (500) eines ersten Satzes von Daten von mindestens einem Sensor von mindestens einer Benutzervorrichtung, wobei der erste Satz Daten mindestens physiologische Daten oder Kontextdaten umfasst;
Sammeln (510) eines zweiten Satzes von Daten, die mindestens eine teilweise oder vollständige Antwort bezüglich einer momentanen ökologischen Einschätzung (EMA) eines Benutzers der mindestens einen Benutzervorrichtung umfassen;
Erstellen (530) eines Satzes von physiologischen Zeitreihendaten für den Benutzer auf Basis des ersten Satzes von Daten und des zweiten Satzes von Daten;
Identifizieren (540) von Gewohnheitsinformationen, die sich auf regelmäßig auftretende Gewohnheiten des Benutzers beziehen, auf Basis des ersten Satzes von Daten;
Detektieren (550) eines potenziellen Auslösepunkts für das Senden einer nachfolgenden EMA an den Benutzer mindestens auf Basis des ersten Satzes von Daten und der Gewohnheitsinformationen, wobei die nachfolgende EMA verwendet wird, um eine Belastungsvalenz des Benutzers zu einer Zeit des potenziellen Auslösepunkts zu ermitteln, wobei das Detektieren des potenziellen Auslösepunkts ferner das Verwenden der Gewohnheitsinformationen, um eine physiologische Erregung, die keinen Bezug zu einer Belastung aufweist, zu ignorieren, umfasst; und
Berechnen (560) einer Schätzung eines Belastungsvalenzwertes, der mit der nachfolgenden EMA verknüpft ist, für die eine EMA-Antwort mindestens teilweise nicht empfangen wurde, zu der Zeit des potenziellen Auslösepunkts, wobei die Schätzung des Belastungsvalenzwertes auf Basis von mindestens einem der physiologischen Zeitreihendaten, der mindestens einen zuvor gesammelten teilweisen oder vollständigen EMA-Antwort, dem ersten Satz von Daten oder den Gewohnheitsinformationen berechnet wird,
wobei das Berechnen der Schätzung des Belastungsvalenzwertes das Durchführen einer Rückverfolgung zum Identifizieren vergangener Trends in den physiologischen Zeitreihendaten des Benutzers umfasst; und
wobei das Durchführen das wiederholte Anwenden eines größer werdenden retrospektiven Zeitfensters umfasst, beginnend bei der Zeit des potenziellen Auslösepunkts, bis eine zuvor erfasste EMA-Antwort gefunden wird, die eine Änderung der physiologischen Daten, der Kontextdaten oder eine beliebige Kombination davon repräsentiert, die der wesentlichen Änderung im ersten Satz von Daten zum potenziellen Auslösepunkt ähnlich ist; und
das Berechnen umfasst ferner das Verwenden einer Belastungsvalenz, die anhand der zuvor erfassten EMA-Antwort bestimmt wurde, als den geschätzten Belastungsvalenzwert für die nachfolgende EMA.

2. Verfahren nach Anspruch 1, wobei der potenzielle Auslösepunkt einen Zeitpunkt umfasst, zu dem der erste Satz von Daten eine wesentliche Änderung des Niveaus zeigt, das über oder unter einem vordefinierten Schwellwert liegt.

3. Verfahren nach einem der Ansprüche 1 bis 2, wobei das Verfahren ferner das Platzieren eines Gewohnheitenankers in den physiologischen Zeitreihendaten, um Zeitperioden zu identifizieren, während denen die regelmäßig auftretenden Gewohnheiten vorkommen, umfasst.

4. Verfahren nach einem der Ansprüche 1 bis 3, wobei nach der Detektion des potenziellen Auslösepunkts das Verfahren ferner das Senden der nachfolgenden EMA an die mindestens eine Benutzervorrichtung und das Berechnen der Schätzung des Belastungsvalenzwertes, der mit der nachfolgenden EMA verknüpft ist, wenn auf die nachfolgende EMA keine Antwort empfangen wird, umfasst.

5. Verfahren nach einem der Ansprüche 1 bis 4, wobei die Kontextdaten mindestens eines von einem geografischen Standort, einer Tageszeit, einer Höhe, einer Außentemperatur oder eine beliebige Kombination davon umfassen.

6. Verfahren nach einem der Ansprüche 1 bis 5, wobei die physiologischen Daten die physischen oder biologischen Eigenschaften des Benutzers umfassen, die mindestens eines von Herzfrequenz, Puls, Kernkörpertemperatur, Atemfrequenz, Blutoxygenierungsniveau, Blutdruck, Hauttemperatur, Herzrhythmus oder eine beliebige Kombination davon beinhalten.

7. Einrichtung, die Folgendes umfasst:
mindestens einen Prozessor; und
mindestens einen Speicher, der einen Computerprogrammcode beinhaltet,
wobei der mindestens eine Speicher und der Computerprogrammcode dazu ausgelegt sind, die Einrichtung mit dem mindestens einen Prozessor mindestens zu Folgendem zu veranlassen
Sammeln (500) eines ersten Satzes von Daten von mindestens einem Sensor von mindestens einer Benutzervorrichtung, wobei der erste Satz Daten mindestens physiologische Daten oder Kontextdaten umfasst;
Sammeln (510) eines zweiten Satzes von Daten, die mindestens eine teilweise oder vollständige Antwort bezüglich einer momentanen ökologischen Einschätzung (EMA) eines Benutzers der mindestens einen Benutzervorrichtung umfassen;
Erstellen (530) eines Satzes von physiologischen Zeitreihendaten für den Benutzer auf Basis des ersten Satzes von Daten und des zweiten Satzes von Daten;
Identifizieren (540) von Gewohnheitsinformationen, die sich auf regelmäßig auftretende Gewohnheiten des Benutzers beziehen, auf Basis des ersten Satzes von Daten;
Detektieren (550) eines potenziellen Auslösepunkts für das Senden einer nachfolgenden EMA an den Benutzer mindestens auf Basis des ersten Satzes von Daten und der Gewohnheitsinformationen, wobei die nachfolgende EMA verwendet wird, um eine Belastungsvalenz des Benutzers zu einer Zeit des potenziellen Auslösepunkts zu ermitteln, wobei das Detektieren des potenziellen Auslösepunkts ferner das Verwenden der Gewohnheitsinformationen, um eine physiologische Erregung, die keinen Bezug zu einer Belastung aufweist, zu ignorieren, umfasst;
Berechnen (560) einer Schätzung eines Belastungsvalenzwertes, der mit der nachfolgenden EMA verknüpft ist, für die eine EMA-Antwort mindestens teilweise nicht empfangen wurde, zu der Zeit des potenziellen Auslösepunkts, wobei die Schätzung des Belastungsvalenzwertes auf Basis von mindestens einem der physiologischen Zeitreihendaten, der mindestens einen zuvor gesammelten teilweisen oder vollständigen EMA-Antwort, dem ersten Satz von Daten oder den Gewohnheitsinformationen berechnet wird; und
Durchführen einer Rückverfolgung, um vergangene Trends in den physiologischen Zeitreihendaten des Benutzers zu identifizieren, um den Belastungsvalenzwert, der mit der nachfolgenden EMA verknüpft ist, zu schätzen, wobei die Rückverfolgung das wiederholte Anwenden eines größer werdenden retrospektiven Zeitfensters umfasst, beginnend bei der Zeit des potenziellen Auslösepunkts, bis eine zuvor erfasste EMA-Antwort gefunden wird, die eine Änderung der physiologischen Daten, der Kontextdaten oder eine beliebige Kombination davon repräsentiert, die der wesentlichen Änderung im ersten Satz von Daten zum potenziellen Auslösepunkt ähnlich ist; und wobei die Belastungsvalenz, die anhand der zuvor erfassten EMA-Antwort bestimmt wurde, als der geschätzte Belastungsvalenzwert für die nachfolgende EMA verwendet wird.

8. Einrichtung nach Anspruch 7, wobei der potenzielle Auslösepunkt einen Zeitpunkt umfasst, zu dem der erste Satz von Daten eine wesentliche Änderung des Niveaus zeigt, das über oder unter einem vordefinierten Schwellwert liegt.

9. Vorrichtung nach einem der Ansprüche 7 und 8, wobei der mindestens eine Speicher und der Computerprogrammcode ferner dazu ausgelegt sind, die Einrichtung mit dem mindestens einen Prozessor zu veranlassen, mindestens einen Gewohnheitenanker in den physiologischen Zeitreihendaten zu platzieren, um Zeitperioden zu identifizieren, während denen die regelmäßig auftretenden Gewohnheiten vorkommen.

## Revendications

1. Procédé mis en œuvre par ordinateur, comprenant :
la collecte (500) d'un premier jeu de données à partir d'au moins un capteur d'au moins un dispositif utilisateur, dans lequel le premier jeu de données comprend au moins l'une de données physiologiques ou de données contextuelles ;
la collecte (510) d'un deuxième jeu de données comprenant au moins une réponse d'évaluation écologique momentanée partielle ou complète (EMA) provenant d'un utilisateur du au moins un dispositif utilisateur ;
l'établissement (530) d'un jeu de données de séries chronologiques physiologiques pour l'utilisateur sur la base du premier jeu de données et du deuxième jeu de données ;
l'identification (540) d'informations d'habitudes concernant des habitudes de l'utilisateur se produisant régulièrement sur la base du premier jeu de données ;
sur la base au moins du premier jeu de données et des informations d'habitudes, la détection (550) d'un point de déclenchement potentiel pour envoyer une EMA ultérieure à l'utilisateur, l'EMA ultérieure étant utilisée pour vérifier une valence de stress de l'utilisateur à un moment du point de déclenchement potentiel, dans lequel la détection du point de déclenchement potentiel comprend en outre l'utilisation des informations d'habitudes pour ne pas tenir compte de l'éveil physiologique non lié au stress ; et
au moment du point de déclenchement potentiel, le calcul (560) d'une estimation d'une valeur de valence de stress associée à l'EMA ultérieure pour laquelle une réponse EMA n'a pas été au moins partiellement reçue, dans lequel l'estimation de valeur de valence de stress est calculée sur la base d'au moins un élément parmi les données de séries chronologiques physiologiques, la au moins une réponse EMA partielle ou complète collectée précédemment, le premier jeu de données, ou les informations d'habitudes,
dans lequel le calcul de l'estimation de la valeur de valence de stress comprend la réalisation d'un retour en arrière pour identifier des tendances passées dans les données de séries chronologiques physiologiques de l'utilisateur ; et
dans lequel la réalisation comprend l'application itérative d'une fenêtre de temps rétrospective croissante commençant à partir du moment du point de déclenchement potentiel jusqu'à la découverte d'une réponse EMA capturée précédemment représentant un changement dans les données physiologiques, les données contextuelles, ou toute combinaison de celles-ci, qui est similaire au changement significatif dans le premier jeu de données au point de déclenchement potentiel ; et
le calcul comprend en outre l'utilisation d'une valence de stress déterminée à partir de la réponse EMA capturée précédemment comme valeur de valence de stress estimée pour l'EMA suivante.

2. Procédé selon la revendication 1, dans lequel le point de déclenchement potentiel comprend un point dans le temps où le premier jeu de données montre un changement significatif de niveau qui est en dessous ou au-dessus d'un seuil prédéfini.

3. Procédé selon l'une quelconque des revendications 1 à 2, le procédé comprenant en outre le placement d'un point d'ancrage d'habitude dans les données de séries chronologiques physiologiques pour identifier des périodes de temps pendant lesquelles les habitudes de l'utilisateur se produisant régulièrement ont lieu.

4. Procédé selon l'une quelconque des revendications 1 à 3, dans lequel, à la détection du point de déclenchement potentiel, le procédé comprend en outre l'envoi de l'EMA ultérieure au au moins un dispositif utilisateur, et le calcul de l'estimation de la valeur de valence de stress associée à l'EMA ultérieure lorsqu'une réponse à l'EMA ultérieure n'est pas reçue.

5. Procédé selon l'une quelconque des revendications 1 à 4, dans lequel les données contextuelles comprennent au moins un élément parmi une localisation géographique, une heure de la journée, une altitude, une température extérieure, ou toute combinaison de celles-ci.

6. Procédé selon l'une quelconque des revendications 1 à 5, dans lequel les données physiologiques comprennent les caractéristiques physiques ou biologiques de l'utilisateur comportant au moins un élément parmi une fréquence cardiaque, un pouls, une température corporelle centrale, une fréquence respiratoire, un niveau d'oxygénation du sang, une pression sanguine, une température de la peau, un rythme cardiaque, ou toute combinaison de ceux-ci.

7. Appareil, comprenant :
au moins un processeur ; et
au moins une mémoire comportant un code de programme informatique,
la au moins une mémoire et le code de programme informatique étant configurés, avec le au moins un processeur, pour amener l'appareil au moins à
collecter (500) un premier jeu de données à partir d'au moins un capteur d'au moins un dispositif utilisateur, dans lequel le premier jeu de données comprend au moins l'une de données physiologiques ou de données contextuelles ;
collecter (510) un deuxième jeu de données comprenant au moins une réponse d'évaluation écologique momentanée partielle ou complète (EMA) provenant d'un utilisateur du au moins un dispositif utilisateur ;
établir (530) un jeu de données de séries chronologiques physiologiques pour l'utilisateur sur la base du premier jeu de données et du deuxième jeu de données ;
identifier (540) des informations d'habitudes concernant des habitudes de l'utilisateur se produisant régulièrement sur la base du premier jeu de données ;
détecter (550), sur la base au moins du premier jeu de données et des informations d'habitudes, un point de déclenchement potentiel pour envoyer une EMA ultérieure à l'utilisateur, l'EMA ultérieure étant utilisée pour vérifier une valence de stress de l'utilisateur à un moment du point de déclenchement potentiel, dans lequel la détection du point de déclenchement potentiel comprend en outre l'utilisation des informations d'habitudes pour ne pas tenir compte de l'éveil physiologique non lié au stress ;
au moment du point de déclenchement potentiel, calculer (560) une estimation d'une valeur de valence de stress associée à l'EMA ultérieure pour laquelle une réponse EMA n'a pas été au moins partiellement reçue, dans lequel l'estimation de valeur de valence de stress est calculée sur la base d'au moins un élément parmi les données de séries chronologiques physiologiques, la au moins une réponse EMA partielle ou complète collectée précédemment, le premier jeu de données, ou les informations d'habitudes ; et
réaliser un retour en arrière pour identifier des tendances passées dans les données de séries chronologiques physiologiques de l'utilisateur afin d'estimer la valeur de valence de stress associée à l'EMA suivante, dans lequel le retour en arrière comprend l'application itérative d'une fenêtre de temps rétrospective croissante commençant à partir du moment du point de déclenchement potentiel jusqu'à la découverte d'une réponse EMA capturée précédemment représentant un changement dans les données physiologiques, les données contextuelles, ou toute combinaison de celles-ci, qui est similaire au changement significatif dans le premier jeu de données au point de déclenchement potentiel, et dans lequel la valence de stress déterminée à partir de la réponse EMA capturée précédemment est utilisée comme valeur de valence de stress estimée pour l'EMA suivante.

8. Appareil selon la revendication 7, dans lequel le point de déclenchement potentiel comprend un point dans le temps où le premier jeu de données montre un changement significatif de niveau qui est en dessous ou au-dessus d'un seuil prédéfini.

9. Appareil selon l'une quelconque des revendications 7 et 8, dans lequel la au moins une mémoire et le code de programme informatique sont en outre configurés, avec le au moins un processeur, pour amener l'appareil au moins à placer un point d'ancrage d'habitude dans les données de séries chronologiques physiologiques pour identifier des périodes de temps pendant lesquelles les habitudes de l'utilisateur se produisant régulièrement ont lieu.
